# NEW EUROPEAN PATENT SPECIFICATION

(11) **EP 1 553 840 B2**
(45) Date of publication and mention of the opposition decision: **25.03.2020**
(45) Mention of the grant of the patent: 03.10.2012
(21) Application number: 03754312.1
(22) Date of filing: 22.10.2003
(51) Int. Cl.: A21D 8/04, A21D 2/02, A21D 2/18, A21D 2/14, C12N 9/98, A23P 10/40, A23L 29/10

(54) **ENCAPSULATED FUNCTIONAL BAKERY INGREDIENTS**
VERKAPSELTE FUNKTIONELLE BACKZUTATEN
INGREDIENTS DE BOULANGERIE FONCTIONNELS ENCAPSULES

(30) Priority: 22.10.2002 EP 02079422
(43) Date of publication of application: 20.07.2005
(73) Proprietor: CSM Nederland B.V., 1112 XE Diemen (NL)
(72) Inventor: DUESTERHOFT, Eva-Maria, NL-6718 ZB Ede (NL); MINOR, Marcel, NL-6704 AA Wageningen (NL); NIKOLAI, Karin, A-9210 Portschach (AT); HARGREAVES, Neil Graham, Chester, CH4 8AE (GB); HUSCROFT, Simon Christopher, NL-2252 TB Voorschoten (NL); SCHARF, Udo, 55413 Weiler (DE)
(74) Representative: Nederlandsch Octrooibureau
(86) International application number: PCT/NL2003/000711
(87) International publication number: WO 2004/037004

(56) References cited:
- EP-A- 0 380 225
- EP-A- 1 008 309
- EP-A1- 0 380 066
- EP-A1- 1 413 202
- WO-A-01/11975
- WO-A-01/25411
- WO-A-02/19828
- WO-A-95/20328
- WO-A-98/32336
- WO-A-99/08553
- GB-A- 1 311 789
- US-A- 2 978 332
- US-A- 3 561 975
- US-A- 3 716 381
- US-A- 4 034 125
- US-A- 4 511 584
- US-B1- 6 312 741
- Wikipedia: Powder Mixture. http://en.wikipedia.org/wiki/Powder_mixtur e, downloaded:17 Juni 2013
- Gaisford et al: Essentials of pharmaceutical preformulation. John Wiley-Blackwell - published: 2013
- Whitaker: Proteolytic Enzymes. In: Whitaker et al. (ed), Handbook of food enzymology, Marcel Dekker 2003 - published: 2003
- D'Alonzo et al: "Glyceride composition of processed fats and oils as determined by glass capillary gas chromatography", JAOCS, vol. 59, no. 7, July 1982 (1982-07), pages 292-295,
- Flack: The role of emulsifiers in low-fat food products. In: Roller et al. (ed): Handbook of Fat Replacers, CRC Press 1996 - published: 1996
- Mahungu et al: Emulsifiers. In: Branen et al. (ed), Food Additives, 2nd ed., Marcel Dekker, Inc., 2001 - published: October 2001
- Kilara et al.: Enzymes. In: Branen et al. (ed), Food Additives, 2nd ed., Marcel Dekker, Inc., 2001 - published: October 2001
- AACC International, Approved Methods of Analysis, 11th ED. Method 58-53.01: Slip Melting Point . published: 1999
- Prescott et al.: On Powder Flow ability. Pharmaceutical Technology, October 2000

## Description

### TECHNICAL FIELD OF THE INVENTION

The present invention is concerned with lipid-encapsulated or lipid-coated functional bakery ingredients, methods for preparing such encapsulated or coated ingredients and the use of these lipid-encapsulated or lipid-coated ingredients in the preparation of a dough composition.

### BACKGROUND OF THE INVENTION

Functional bakery ingredients are widely used in the baking industry to improve handling and machinability of doughs and also to improve texture, volume, flavour, and freshness (anti-staling) of the final baked product. Examples of functional bakery ingredients that can be used to "condition" a dough include enzymes, oxidoreductants, acidulants, hydrocolloids, starches, yeast, sugars, water and flavours.

An important area of application of functional bakery ingredients is bread. Bread is made from four principal ingredients: flour, yeast, salt and water. It is usually prepared in three basic steps, and the end result is a baked loaf. The steps are: (a) the principal ingredients are mixed to form a dough and worked to develop a continuous visco-elastic gluten matrix; (b) the developed dough is then proved by incubation in warm, humid conditions to promote fermentation by the yeast causing the dough to rise; (c) the risen dough is then baked to gelatinise starch, denature protein and fix the dough structure. Various additives, including the aforementioned functional bakery ingredients, are known to improve dough development and the quality of the baked loaf. These additives are generally known as bread (or flour or dough) improvers/conditioners.

The strength of a dough is an important aspect of baking for both small-scale and large-scale applications. A strong dough has a greater tolerance of mixing time, proving time, and mechanical vibrations during dough transport, whereas a weak dough is less tolerant to these treatments. A strong dough with superior rheological and handling properties results from flour containing a strong gluten network. Flour with a low protein content or a poor gluten quality results in a weak dough.

Non-specific oxidants, such as iodates, peroxides, ascorbic acid, potassium bromate, glutathione and azodicarbonamide have a gluten strengthening effect. It has been suggested that these dough improvers induce the formation of interprotein bonds which strengthen the gluten and thereby the dough. The use of several of the currently available chemical oxidising agents has been met with consumer resistance or is not permitted by regulatory agencies.

The use of enzymes as dough improvers has been considered as an alternative to the chemical conditioners. A number of enzymes have been used recently as dough and/or bread improving agents, in particular enzymes that act on components present in large amounts in the dough. Examples of such enzymes are found within the groups of amylases, xylanases, proteases, glucose oxidases, oxygenases, oxidoreductases, trans-glutaminases and (hemi) cellulases, including pentosanases.

The use of the aforementioned dough improvers is not uncomplicated, since these functional ingredients tend to affect dough properties such as stickiness, strength and/or stability. As a result, the dough can become difficult to handle both by hand and by machines. It would thus be desirable to be able to delay the moment when the conditioner exerts its full functionality until after a selected point in time. In particular, it would be desirable to delay such a moment until all dough ingredients have been mixed and especially until such time that proving of said dough has commenced.

The lipid-encapsulation or lipid-coating of food ingredients to prevent functional ingredients from exerting their functionality prematurely is known in the art. US 3,561,975 describes a pie crust shrinkage-reduction agent which maintains good handling properties before baking, said agent consisting of substantially spherical particles each comprising shortening having embedded regularly therein proteolytic enzyme particles, said spherical particles having diameters ranging from about 150 microns to about 1.5 millimeters, said shortening comprising triglyceride having a complete melting point from about 95° to about 155°F (35.0-68.3°C), the weight ratio of said shortening to said enzyme ranging from about 20 to 1 to about 1 to 1. The US-patent furthermore discloses the incorporation of sorbitan fatty glyceride polyoxyethylene derivatives (Tween ®) in an amount of 11% by weight of the triglyceride. The enzyme particles within the spherical particles are said to have a longest dimension ranging from about 5 to about 150 microns, preferably ranging from about 10 to about 50 microns.

DE-A 2 203 429 is concerned with a process for the preparation of an acid composition that displays delayed dissolving behaviour, wherein a solid acid or an acid contained in a solid carrier is coated with an edible fat that is solid at ambient temperature and that contains an emulsifier. The melting point of the fat is in the range of 45°-60°C. It is stated that the emulsifier may be soy lecithin, 0.1-10% glycerol monostearate or 1-20% glycerol polyricinoleate. The acid compositions described in the German patent application are particularly useful for application in yoghurt.

EP-A 0 380 066 describes particles containing a water-soluble core and a coating that contains high melting fat, wax, lecithin and fatty acid. The possibility of including enzymes in the water-soluble core is mentioned. The preferred particle size is said to be in the range of 150-250 microns. The lipid coating of the particles contain 0.05-1.2% wax, 0.01-0.05% lecithin and 0.01-5% fatty acids by weight of fat. The European patent application mentions the use of the coated particles in flour products. Specific examples mentioned are frying batter, tempura coatings and frying flour.

US 3,716,381 describes a method of preserving meat and fish products subjected to heat treatment in a final finishing which comprises adding to the raw meat or fish a granular preservative comprising sorbic acid powder particles whose surface has been coated with a hardened oil having a melting temperature of 40°-90°C. It is observed in the US-patent that a small amount of a surfactant for food use, such as glycerol monostearate or acetylated monoglyceride, can be used along with the hardened oil.

EP 1 008 309 discloses a granular baking improver with a particle size of 300-500 micrometers. A powdered premix (sugar, acidulant, enzymes) is coated with a mixture of emulsifier and malt extract or high melting fat.

WO 99/08553 discloses granulated bakery ingredients coated with fatty substances selected from glycerides and emulsifiers. The substances are used during bread preparation.

WO 98/32336 and WO 01/11975 discloses fat (WO 98/32336: slip melting point 35°C; WO 01/11975 preferably triglyceride) coated enzyme granules, in WO 98/32336 granules with a size of around 150 micrometer. Doughs are prepared.

WO 02/19828 discloses enzymes coated by a lipid layer having a slip melting point of 25°-60°C). They are mixed with conventional dough ingredients.

US 3 716 381 discloses fat/emulsifier coated sorbic acid particles having a particle size of 50-300 micrometer. The particles are integrated into fish/meat sausage mixtures.

US 6 312 741 discloses fumaric acid coated with a lipid coating having a melting point within normal baking temperature selected from lipid materials. Tortillas are prepared.

GB 1 311 789 discloses mono/diglyceride/hardened oil coated acid coated particles having a size of 50-500 micrometer. Bread doughs are prepared.

US 4 034 125 discloses a bakery powder comprising dispersed acetic acid/lactic acid coated with fatty acid glyceride material and adsorbed in starch polysaccharide or cellulosic material. The particles are added to sourdough mixtures, followed by the addition of water.

It is an object of the present invention to provide improved lipid-encapsulated or lipid-coated functional bakery ingredient(s) that are relatively stable under ambient conditions and which at the same time release the functional bakery ingredient rapidly in a controlled manner when said functionality is required, especially during proving of the dough.

### SUMMARY OF THE INVENTION

The inventors have discovered that the aforementioned objective is met by a composition comprising granules suitable for use in the preparation of a dough, said granules having an average diameter in the range of 30-500 µm and comprising:
a. a hydrophilic core with a diameter of at least 5 µm, said core containing one or more functional bakery ingredients selected from enzymes; and
b. a lipophilic substantially continuous layer encapsulating the core, which layer contains at least 50 wt.% triglyceride fat with a slip melting point of at least 30°C and at least 1 wt.% of a release agent selected from the group of monoglycerides, diacetyl tartaric acid ester of mono- and/or diglyceride (datem), stearyl-lactylates and combinations thereof;
and further comprising one or more bakery ingredients in particulate form, said one or more bakery ingredients being selected from the group consisting of redox agents, emulsifiers, hydrocolloids, flour, salts, malt flour, malt extract, gluten and starch; said composition displaying free flowing behaviour.

Although the inventors do not wish to be bound by theory, it is believed that the aforementioned release agents enable the controlled release of the functional bakery ingredient(s) after the granules have been incorporated in the dough and in particular that they enable a release that increases rapidly with increasing temperature. Thus, granules according to the invention offer the advantage that they protect the functional bakery ingredient during storage and transport. In addition, unlike unencapsulated or uncoated functional ingredients, they make available the functionality of these functional ingredients in a controlled way during the dough preparation process, which clearly improves the handling properties of the dough. As compared to the coated and encapsulated systems known from the prior art, the present granules offer the advantage that the functionality is generally released in a more gradual way, allowing the functional ingredient to already exert some of its functionality early on during the dough preparation process. In case of enzymes, for instance, such an early controlled action is desired to produce a baked product with good consistency and volume. Thus, the invention enables the preparation of a dough that is easy to handle and that yields a baked product with excellent consistency and volume.

The release agents employed in accordance with the present invention are also used as emulsifiers in a variety of food products. The inventors have found, however, that other emulsifiers, when used to substitute the release agent in the present granule, do not enhance the release of the functional bakery ingredient under the above mentioned conditions. Thus, the release enhancing properties of the present release agents are not common to emulsifiers.

### DETAILED DESCRIPTION OF THE INVENTION

Accordingly, one aspect of the present invention is concerned with a composition comprising granules suitable for use in the preparation of a dough, said granules having an average diameter in the range of 30-500 µm and comprising:
a. a hydrophilic core with a diameter of at least 5 µm, said core containing one or more functional bakery ingredients selected from enzymes; and
b. a lipophilic substantially continuous layer encapsulating the core, which layer contains at least 50 wt.% triglyceride fat with a slip melting point of at least 30°C and at least 1 wt.% of a release agent selected from the group of monoglycerides, diacetyl tartaric acid ester of mono- and/or diglyceride (datem), stearyl-lactylates and combinations thereof;
and further comprising one or more bakery ingredients in particulate form, said one or more bakery ingredients being selected from the group consisting of redox agents, emulsifiers, hydrocolloids, flour, salts, malt flour, malt extract, gluten and starch; said composition displaying free flowing behaviour.

The term "slip melting point" is defined as the temperature at which the amount of solid phase in the melting fat has become so low that an air bubble is forced upwards in an open capillary filled with the fat.

It is noted that the granules according to the present invention may take the form of a single hydrophilic core that is enveloped by a lipophilic substantially continuous layer. Alternatively, the granules may comprise two or more hydrophilic cores that are each enveloped by a lipophilic substantially continuous layer. The latter granules may suitably be obtained, for instance, by means of spray chilling, as will be described below.

The positive impact of the above mentioned release agents is believed to be associated with their surface activity and in particular their ability to enhance the formation of a large oil-water interface once a significant part of the triglyceride fat has melted. In particular monoglycerides, datem and/or stearyl lactylates may advantageously be incorporated in the lipophilic layer of the granules of the present invention. Even more preferably, the release agent is selected from the group consisting of monoglycerides, datem and combinations thereof. In one particularly preferred embodiment of the invention the release agent is monoglyceride. In another preferred embodiment, the release agent is stearyl lactylate.

The release agent employed in accordance with the present invention preferably contains one or more fatty acid residues with, on average, 4-24 carbon atoms. Such release agents will usually display a slip melting point between 5 and 80 °C. More preferably the slip melting point of such a release agent is within the range of 20 and 70°C. Most preferably the slip melting point exceeds 30 °C.

The lipophilic substantially continuous layer preferably contains from 50-98 wt.% of triglyceride fat and from 2-50 wt.% of the release agent. More preferably the lipophilic layer contains from 60-94 wt.% triglyceride fat, most preferably from 70-92 wt.%. The amount of release agent within the lipophilic layer preferably is at least 2 wt.%, more preferably it is at least 3 wt.% and most preferably it is at least 4 wt.%. Typically, the amount of release agent within the lipophilic layer is not more than 40 wt.%, preferably not more than 30 wt.% and most preferably not more than 25 wt.%. Typical bakery enzymes that are advantageously incorporated include α-amylase, β-amylase, xylanase, hemi-cellulase, cellulase, lipase, protease, glucose oxidase, hexose oxidase, oxidoreductase, lipoxygenase, peroxidase, ferulic acid esterase, pullulanase, invertase, mannanase, galactomannanase, lactase and combinations thereof. Preferably the bakery enzyme is selected from the group consisting of α-amylase, xylanase and combinations thereof. Most preferably, the bakery enzyme is α-amylase.

As explained herein before, the present invention offers the advantage that the impact of the functional ingredient(s) on the dough is delayed, e.g. until the start of the proving process. Thus, these functional ingredients exert a considerable part of their desired effect during or after proving, thereby avoiding or reducing problems with e.g. stickiness, water holding capacity and dough strength. As regards the aforementioned hydrocolloids it is also advantageous to delay the thickening/gelling effect of these hydrocolloids until after the start of the proving process. If the hydrocolloids start to exert their effect during the admixing of the principal dough components, a relatively viscous mass is obtained that is difficult to handle. In case a gel-forming hydrocolloid is used, the actual mixing operation and/or subsequent handling may disrupt the gel-structure, thereby annihilating the desired functionality of such a gelling hydrocolloid.

The functional bakery ingredient in the present granule is an enzyme. The term enzyme as used in here refers to any preparation of enzyme at any level of purity, so long as the preparation is enzymatically active. The term enzyme also encompasses a preparation exhibiting a plurality of different specific enzymatic activities.

The benefits of the present invention are particularly pronounced if the functional bakery ingredient(s) are mainly released from the granule during the proving step rather than during the preceding mixing or the subsequent baking step. In order to achieve this, it is preferred to employ a triglyceride fat displaying a slip melting point in the range of 30-40°C. More preferably the triglyceride fat has slip melting point in the range of 33-40°C. Most preferably the slip melting point is in the range of 34-38°C. In particular in case the functional bakery ingredient(s) comprise one or more enzymes, it is very advantageous to design the lipophilic layer in such a way that substantially all of the encapsulated enzyme is released during proving as the activity of most enzymes will decline rapidly during the course of the baking process.

In a preferred embodiment, the lipophilic layer, comprising the combination of triglyceride fat and release agent, has a slip melting point in the range of 30-40°C, more preferably in the range of 33-40°C and most preferably in the range of 34-38°C.

As explained above, the granules of the present invention may advantageously be applied in doughs to achieve a controlled release of the functional bakery ingredient(s). The granules of the invention combine the capacity to delay the release of the one or more functional ingredients with the ability to release these ingredients within a relatively short time interval. Since the duration of the proving can be rather short (e.g. about 15-20 minutes) the swift release of functional ingredients that are meant to exert their effect during proving is very advantageous.

In order to facilitate the swift release of the one or more bakery ingredients, it was found to be advantageous to additionally incorporate into the core of the granule a hygroscopic component. Usually such a hygroscopic component is incorporated in a weight ratio of hygroscopic component: functional bakery ingredient(s) in the range of 1:2 to 20:1, preferably of 1:1 to 10:1. Examples of hygroscopic components that may suitably be used to accelerate the release of the functional ingredient(s) include xanthan gum, guar gum, locust been gum, carrageenan, alginate, pectin, CMC, HPMC, starches, dextrins, sugar, salts and combinations of these components. It is noted that, although the hygroscopic component may be a functional bakery ingredient, in accordance with the present invention the hygroscopic component is not an enzyme, an oxidoreductant, an acidulant, or yeast. Particularly preferred are hygroscopic components that swell as a result of absorption of water, especially thickening and gelling agents. Since the formation of a gel structure may hinder the effective release of the functional ingredients, the present granule most preferably contains a thickening agent, e.g. guar gum or locust bean gum.

Like the release agent and the optional hygroscopic component, also the triglyceride fat employed in the present granule has an important impact on the release characteristics. The triglyceride fat also has an important impact on the stability of the granule, especially during storage and handling. It is important that the lipophilic layer is strong enough to withstand handling and mixing. In addition the lipophilic layer should not be sticky as otherwise the granules will agglomerate which will hamper dosing of the granules. In order to enable the preparation of granules that are free flowing, that are storage stable and that survive normal mixing operations, it is advantageous to employ a triglyceride fat that displays an N-profile of N₂₀ > 50; 10 ≤ N₃₀ ≤ 60; and N₄₀ < 5. Preferably the triglyceride fat displays an N₃₀ <50, even more preferably an N₃₀ < 40; and an N₄₀ < 2. Furthermore, the triglyceride fat preferably displays an N₃₀ > 20, more preferably an N₃₀ > 30. The N-profile refers to the solid fat content in the triglyceride fat at the indicated temperature (N₄₀ refers to the solid fat content at 40°C) and is determined by means of pulse NMR.

The triglyceride fat in the lipophilic layer may comprise unmodified, hydrogenated, fractionated and/or interesterified triglycerides. Examples of particularly suitable triglyceride fats include palm mid fractions, palm kernel stearine, cocoa butter and butteroil stearine. Preferably the triglyceride fat contains at least 50 wt.%, more preferably at least 80 wt.% of one or more of these fats or interesterified blends of these fats.

In order to achieve highly desirable release characteristics, it is recommendable that the core constitutes between 10 and 99 wt.% of the granule. More preferably the core constitutes between 20 and 95 wt.% of the granule. In case the granule comprises more than one core, the latter percentages refer to the total amount of core material contained within the granule.

The core(s) within the present granule preferably has a diameter of at least 30 µm, more preferably of at least 50 µm. Generally the diameter of the core will not exceed 800 µm, more preferably it will not exceed 500 µm and most preferably it will not exceed 200 µm. The lipophilic layer will usually have a thickness of at least 2 µm, preferably at least 5 µm, most preferably of at least 10 µm. Normally the thickness of the lipophilic layer will not exceed 200 µm. Preferably the thickness of said layer does not exceed 100 µm, more preferably it does not exceed 70 µm. In case the granule comprises two or more cores, the thickness of the lipophilic layer, in as far as it does not form an interface between core and environment, is defined by the distance between the cores, excluding interstitial spaces.

In order to further stabilise the present granule and also to improve its free flowing characteristics, it can be advantageous to apply an additional exterior coating containing at least 50 wt.% of an agent selected from the group consisting of sugar, dextrin, tricalciumphosphate, silicate, calcium carbonate and combinations thereof. More preferably said coating contains at least 70 wt.%, most preferably it contains at least 80 wt.% of such an agent.

The granule of the present invention suitably has a diameter in the range of 10-1000 µm, preferably of 30-500 µm and more preferably of 60-400 µm. Most preferably the diameter of the present granule is within the range of 80-300 µm.

Another aspect of the invention relates to a composition comprising granules as described above, wherein the average diameter of the granules is in the range of 10-1000 µm, more preferably within the range of 30-500 µm and most preferably within the range of 60-300 µm. In order to achieve desirable release characteristics it is advantageous that the particle size distribution of the granules is relatively narrow. Typically, at least 90% of the particles has a particle size within the range of 20-300 µm, more preferably within the range of 30-200 µm and most preferably within the range of 40-100 µm.

As mentioned herein before the present granules and granule containing compositions of the invention exhibit the highly advantageous property that they quickly release the functional bakery ingredient(s) contained therein above a certain temperature in the presence of water. Typically, at least 50 wt.% of the functional bakery ingredient contained in the granule is released within 10 minutes when said granule is immersed in water of a temperature of 38°C. In order to test whether a granule containing composition meets this criterion, a sample equivalent to 75 mg core material should be introduced in a test tube containing 15 ml buffer (0.05M sodium acetate, pH 5.2) at 7 °C. The tube is gently rotated head over tail for 10 minutes at 7 °C. Subsequently, the tube is immersed in a water batch of 38 °C for 10 minutes. After the 10 minutes have passed, the tube is quickly cooled in ice-water. Next, the contents of the tube are immediately passed through a funnel filled with glass wool. The released amount of the functional bakery ingredient is calculated by dividing the amount recovered in the filtered liquid by the total amount present in the original sample.

The present composition contains a combination of granules as defined herein before and other bakery ingredients. These ingredients are also in a particulate form so that the total composition displays free flowing behaviour. Examples of additional bakery ingredients that may be incorporated in the present composition include redox agents, emulsifiers, hydrocolloids, flour, salts, malt flour, malt extract, gluten and starch.

Yet another aspect of the invention relates to the use of the aforementioned composition in the preparation of a dough, preferably in the preparation of a bread dough. The dough may simply be prepared by mixing the present composition with the other dough components, e.g. flour, water and yeast. Usually the present composition is incorporated in an amount of between 0.01 and 5% by weight of the dough.

The invention also encompasses a method for the preparation of granules as described above. The present granules may be prepared by a variety of processes including, for example, fluidised bed coating and spray chilling, fluidised bed coating being most preferred.

In a preferred embodiment, the present method comprises the steps of:
a. preparing a plurality of particles with a diameter of at least 5 µm, said particles containing one or more functional bakery ingredients selected from enzymes;
b. preparing a blend containing at least 50 wt.% of a triglyceride fat with a slip melting point of at least 30°C and at least 1 wt.% of a release agent selected from the group of monoglycerides, datem, stearyl-lactylates and combinations thereof; and
c. spraying the blend obtained from step b. in melted form onto the plurality of particles obtained from step a. to achieve encapsulation of the particles with a substantially continuous layer of the said blend; and
d. cooling the resulting encapsulated particles to obtain a plurality of encapsulated particles that exhibit free flowing behaviour.

In step c., when the blend is sprayed onto the plurality of particles, said particles preferably have a temperature that is significantly lower than the slip melting point of the blend. Thus, the blend will start to solidify onto said particles, making it easier to maintain the fluidised bed conditions during the coating process. Preferably the temperature of the particles is at least 2 °C, more preferably at least 5 °C below the slip melting point of the blend. At the same time, the temperature of the particles preferably is not more than 30 °C, more preferably not more than 20 °C, more preferablyt not more than 15 °C below the slip melting point of the blend.

In a particularly preferred embodiment the plurality of particles is prepared in step a. by spray drying, preferably by spray drying the functional bakery ingredient(s) together with a hygroscopic component as defined herein before.

The present invention also encompasses a method of manufacturing granules and compositions as defined herein before, said method comprising the steps of:
a. preparing a plurality of particles with a diameter of at least 5 µm, said particles containing one or more functional bakery ingredients selected from enzymes;
b. combining the plurality of particles with triglyceride fat and a release agent selected from the group of monoglycerides, diacetyl tartaric acid ester of mono- and/or diglyceride (datem), stearyl-lactylates and combinations thereof to provide a blend wherein the lipophilic component contains at least 50 wt.% triglyceride fat with a slip melting point of at least 30°C and at least 1 wt.% of the release agent;
c. preparing a homogeneous suspension from the blend obtained from step b., wherein the continuous phase of the suspension is formed by molten lipophilic component;
d. atomising the homogeneous suspension into a gaseous or liquid medium with a temperature below the melting point of the combination of the lipophilic component; and
e. recovering the resulting granules.

It is noted that the sequence of combining triglyceride fat, release agent and particles is not critical. Also, the suspension comprising molten lipophilic component may be provided using different approaches, e.g. by dispersing the particles into molten triglyceride fat and/or molten release agent, or by blending granulated triglyceride fat, with the particles and/or the release agent followed by melting.

The medium that is used to solidify the molten components may suitably consist of a gas or liquid. Preferably, said medium has a temperature that is at least 3°C, more preferably at least 8°C and most preferably at least 15°C below the slip melting point of the aforementioned combination. In a particularly preferred embodiment, the medium is a gas, in particular air or nitrogen, air being most preferred.

The invention is further illustrated by means of the following examples.

### EXAMPLES

### Example 1

Fungamyl^{®} 1600 bakery granulate (a commercial α-amylase preparation obtained from Aspergillus oryzae; Novo Nordisk) is coated on a fluidised bed laboratory unit (GPCG 1.1, Glatt) with Wurster geometry.

Fungamyl 1600 is fluidised by air. A fat blend consisting of 90 wt.% of a hydrogenated stearin fraction of palm kernel oil (slip melting point of 35°C) and 10 wt.% distilled monoglyceride (Monomuls 90 ex Cognis™; melting point of about 40°C) is molten and sprayed onto the fluidised granulate. Airflow, bed temperature, fat temperature and flow rate, atomisation air pressure and temperature are controlled in such a way that a closed fat film around the granulate particle is formed. The bed temperature is maintained at a sufficiently high temperature to prevent that the fat solidifies before wetting the granulate, leading to free fat particles and uncoated granulate, and a sufficiently low temperature to prevent the bed from agglomerating as a results of the formation of sticky particles.

Two experiments are carried out in which the granulate is coated with different amounts of the fat blend. The amount of fat in the final product is determined by means of low resolution NMR. In both cases the value so obtained is in close agreement with the one calculated from the total amount of fat that was sprayed onto the granulate. The two encapsulates obtained are found to contain approximately 50 wt.% or 75 wt.% of the fat blend.

The particle size distribution is measured via static light scattering (Malvern™ 2600C). The uncoated enzyme granulate exhibits an average diameter of about 150 µm. The coated granules containing about 50 wt.% fat blend display an average diameter of about 310 µm, whereas the coated granules containing about 75 wt.% of the same blend display an average diameter of about 490 µm.

The stability of the coated granules in an aqueous environment is assessed by suspending the granules containing equivalent amounts of enzyme in demineralised water of 24°C and measuring the electric conductivity as a function of time. The curves obtained show a rapid increase in conductivity that flattens off within 2-10 minutes, indicating that in both cases a minor amount of the granulate has not been encapsulated perfectly. The granules containing 50 wt.% of fat blend show a significantly faster initial release than the granules containing 75 wt.% of fat blend. The plateau in the curves is achieved at a significantly lower conductivity for the 75 wt.% product than the 50 wt.% product, indicating that the encapsulation with 75 wt.% fat blend is more effective.

The coated granules are again suspended in demineralised water of 24°C and the electric conductivity is measured while the temperature of the water is increased at a rate of about 3 °C/minute. In both cases a sharp increase in conductivity is observed at a temperature close to the melting point of the fat.

The temperature dependency of the release characteristics of the coated granules containing 75 wt.% fat blend is determined by suspending 300 mg of these granules in 4 different tubes containing 15 ml of an aqueous buffer (0.05M sodium acetate buffer, pH 5.2) at 7°C; and 75 mg of the original uncoated granulate in another tube containing 15 ml of the same buffer (control). The tubes are gently rotated head over tail at 7°C for 10 min. Then, the tubes are immersed in different water-baths of 25, 30, 35 and 45°C respectively. After 15 minutes in the water bath, each suspension is quickly cooled in ice-water. Subsequently, the suspensions are centrifuged and filtered and the enzyme activity in the filtered solution is measured. Results show that the suspension that was kept in a water bath at 45°C exhibits the same enzyme activity as the control sample (kept under the same conditions), meaning that effectively all of the encapsulated enzyme was released. Furthermore, it is found that, in the suspension that was kept at 35°C, a major fraction of the enzyme activity has been released. The other 2 suspensions, i.e. those that were kept at 30 and 25°C, only release a minor fraction of the enzyme activity during equilibration at these elevated temperatures.

### Example 2

The α-amylase preparation of Example 1 was coated by means of spray chilling. Six different lipid coatings were applied to the enzyme preparation:

| | **Release agent** | **Composition lipid coating** |
|---|---|---|
| Granulate 1 | None | 100% triglyceride fat |
| Granulate 2 | Monoglycerides | 95% triglyceride fat / 5% release agent |
| Granulate 3 | Monoglycerides | 90% triglyceride fat / 10% release agent |
| Granulate 4 | Stearyl lactylate | 95% triglyceride fat / 5% release agent |
| Granulate 5 | Stearyl lactylate | 90% triglyceride fat / 10% release agent |
| Granulate 6 | Datem | 90% triglyceride fat / 10% release agent |

The triglyceride fat was a hydrogenated stearin fraction of palm kernel oil (slip melting point of 35°C).The monoglyceride employed was the same as described in Exampe 1. The stearyl lactylate employed was SSL P 55 VEG ex Danisco™ (melting point 45°C). The datem product used was Panodan AB 100 FS/C ex Danisco™.

In order to obtain a fine powder Fungamyl^{®} 1600 bakery granulate was milled to a particle size of D [v,0.5] = 60µm. The particle size was determined via light scattering (Malvern™ 2600c). The lipid coating material was molten by heating to 65°C. Subsequently, 900 g of the molten material was taken and 100 gram of the milled Fungamyl 1600 was dispersed therein with the help of an Ultra Turrax ®. The temperature of the dispersion was monitored with a digital thermometer and kept constant at 65°C.

Atomisation of the dispersion was performed with a heatable two-fluid spray nozzle. Water from a thermostated water bath (65°C) was pumped through the nozzle, keeping the nozzle at constant temperature well above the melting point of the fat, preventing premature congealing in the nozzle. The fat dispersion was transported via a syringe pump through heated tubes towards the nozzle and atomized by nitrogen gas under pressure. The particle size of the atomized fat can be adjusted in a range of 40 - 2000 µm by changing the atomisation pressure. The droplets were sprayed into liquid nitrogen and collected at the end of the process by simply evaporating the residual liquid nitrogen. The particle powder was then sieved into a fraction with particle sizes of 200 - 400 µm and a fraction with particles sizes of 400 - 800 µm.

The release properties of the coated granules in an aqueous environment were determined by suspending a small amount of the granules in demineralised water of 20°C and measuring the electric conductivity as a function of time for several hours. The particle fractions 200 - 400 µm were taken for the measurement. The conductivity for 100% release was determined by heating the water well above the melting point of the capsule, cooling back to 20°C and measuring the conductivity. Thus, the release curves (in % release) can be calculated.

For granulates 2, 3 and 6 a steep increase in conductivity is observed which flattens of over time. The conductivity measured for granulates 4 and 5 increases at a significantly slower rate. The rate of conductivity increase observed for granulate 1, i.e. the granulate coated with a lipid that does not contain any release agent, is much lower than observed for any of the other granulates.

The above experiments were repeated with the exception that instead of measuring the conductivity an assay was used to determine the enzyme activity that was released from the granules over time. The results obtained corresponded well with the results obtained from the conductivity measurements.

### Example 3

Five different bread doughs are prepared on the basis of the recipes presented in the table below. All five doughs contain 60 mg amylase preparation (Fungamyl ex Novo™). Doughs 2, 3, 4 and 5 are prepared by incorporating therein fat coated amylase granules (10% amylase and 90% lipid coating) that are prepared as described in example 2. The granules used in dough 2 contain a fat coating consisting of a triglyceride fat with a slip melting point of 34°C. The granules incorporated in doughs 3, 4 and 5 contain the same triglyceride fat in combination with a release agent in accordance with the present invention (the same as described in Example 2). The granules used in dough 3 contain 10% monoglycerides by weight of the fat coating. Dough 4 contains 10% stearyl lactylate by weight of the fat coating. Dough 5 contains 10% datem by weight of the fat coating. The processing conditions used in the preparation of the doughs and the breads baked therefrom are also depicted in the table below.

| | ***Dough 1*** | ***Dough 2*** | ***Dough 3*** | ***Dough 4*** | ***Dough 5*** |
|---|---|---|---|---|---|
| ***Recipe:*** | | | | | |
| *Wheatflour (g)* | *3000* | *3000* | *3000* | *3000* | *3000* |
| *Water (g)* | *1740* | *1740* | *1740* | *1740* | *1740* |
| *Yeast (g)* | *150* | *150* | *150* | *150* | *150* |
| *Salt (g)* | *60* | *60* | *60* | *60* | *60* |
| *Ascorbic acid (mg)* | *225* | *225* | *225* | *225* | *225* |
| *Xylanase (mg)* | *150* | *150* | *150* | *150* | *150* |
| *Amylase Prep. (mg)* | *60* | --- | --- | --- | - |
| *Amylase Encap. (mg)* | --- | *600* | --- | --- | --- |
| *Amlyase Encap.*/*Mg (mg)* | --- | --- | *600* | --- | --- |
| *Amylase Encap.*/*SSL (mg)* | --- | --- | --- | *600* | --- |
| *Amlyase Encap*/*Datem (mg)* | --- | --- | --- | --- | *600* |
| | | | | | |

| ***Process:*** | | | | | |
|---|---|---|---|---|---|
| *Mixing time (spiral) (min.)* | *2*+*5* | *2*+*5* | *2*+*5* | *2*+*5* | *2*+*5* |
| *Dough rest (min.)* | *0* | *0* | *0* | *0* | *0* |
| *Floor time (min.)* | *15* | *15* | *15* | *15* | *15* |
| *Proof time (min.)* | *35* | *35* | *35* | *35* | *35* |
| *Baking time (min.)* | *20* | *20* | *20* | *20* | *20* |
| *Baking temp. (° C)* | *240* | *240* | *240* | *240* | *240* |

During dough preparation it is observed that dough 1 is more sticky and more difficult to handle than the other doughs, presumably as a result of enzyme activity during the dough preparation stage. The baked breads obtained from the aforementioned doughs are evaluated by an expert panel. It is found that in terms of dough consistency and specific volume, the baked products obtained from doughs 1, 3, 4 and 5 are quite similar, be it that the product obtained from dough 4 is found to exhibit a slightly less elastic consistency. The product obtained from dough 2 is found to have a much more dry and stiff consistency than the other baked products. Also the specific volume of this product is found to be significantly lower than that of the other products.

## Claims

1. A composition comprising granules suitable for use in the preparation of a dough, said granules having an average diameter in the range of 30-500 µm and comprising:
a. a hydrophilic core with a diameter of at least 5 µm, said core containing one or more functional bakery ingredients selected from enzymes; and
b. a lipophilic substantially continuous layer encapsulating the core, which layer contains at least 50 wt.% triglyceride fat with a slip melting point of at least 30°C and at least 1 wt.% of a release agent selected from the group of monoglycerides, diacetyl tartaric acid ester of mono- and/or diglyceride (datem), stearyllactylates and combinations thereof;
and further comprising one or more bakery ingredients in particulate form, said one or more bakery ingredients being selected from the group consisting of redox agents, emulsifiers, hydrocolloids, flour, salts, malt flour, malt extract, gluten and starch;
said composition displaying free flowing behaviour.

2. The composition according to claim 1, wherein the core contains an enzyme selected from the group consisting of α-amylase, β-amylase, xylanase, hemi-cellulase, cellulase, lipase, protease, glucose oxidase, oxidoreductase, lipoxygenase, peroxidase, ferulic acid esterase, pullulanase, invertase, mannanase, galactomannanase, lactase and combinations thereof.

3. The composition according to claim 1, wherein the release agent is monoglyceride.

4. The composition according to claim 1, wherein the release agent is datem.

5. The composition according to any one of the preceding claims, wherein the lipophilic layer contains between 2 and 40 wt.% of the release agent.

6. The composition according to any one of the preceding claims, wherein the triglyceride fat displays a slip melting point in the range of 30-40°C.

7. The composition according to any one of the preceding claims, wherein the triglyceride fat displays an N-profile of N₂₀ > 50; 10 ≤ N₃₀ ≤ 60; and N₄₀ < 5.

8. The composition according to any one of the preceding claims, wherein the average diameter of the granules is in the range of 60-400 µm.

9. Use of the composition according to any one of the preceding claims in the preparation of a dough, preferably a bread dough.

10. A method of manufacturing a composition according to any one of claims 1-8, said method comprising the steps of:
a. preparing a plurality of particles with a diameter of at least 5 µm, said particles containing one or more bakery ingredients selected from enzymes;
b. preparing a blend containing at least 50 wt.% of a triglyceride fat with a slip melting point of at least 30°C and at least 1 wt.% of a release agent selected from the group of monoglycerides, diacetyl tartaric acid ester of mono- and/or diglyceride (datem), stearyl-lactylates and combinations thereof; and
c. spraying the blend obtained from step b. in melted form onto the plurality of particles obtained from step a. to achieve encapsulation of the particles with a substantially continuous layer of the said blend;
d. cooling the resulting encapsulated particles to obtain a plurality of encapsulated particles that exhibit free flowing behaviour; and
e. incorporating one or more bakery ingredients in particulate form, said one or more bakery ingredients being selected from the group consisting of redox agents, emulsifiers, hydrocolloids, flour, salts, malt flour, malt extract, gluten and starch.

11. A method of manufacturing a composition according to any one of claims 1-8, said method comprising the steps of:
a. preparing a plurality of particles with a diameter of at least 5 µm, said particles containing one or more bakery ingredients selected from enzymes;
b. combining the plurality of particles with triglyceride fat and a release agent selected from the group of monoglycerides, diacetyl tartaric acid ester of mono- and/or diglyceride (datem), stearyl-lactylates and combinations thereof to provide a blend wherein the lipophilic component contains at least 50 wt.% triglyceride fat with a slip melting point of at least 30°C and at least 1 wt.% of the release agent;
c. preparing a homogeneous suspension from the blend obtained from step b., wherein the continuous phase of the suspension is formed by molten lipophilic component;
d. atomising the homogeneous suspension into a gaseous or liquid medium with a temperature below the melting point of the lipophilic component; and
e. recovering the resulting granules; and
f. incorporating one or more bakery ingredients in particulate form, said one or more bakery ingredients being selected from the group consisting of redox agents, emulsifiers, hydrocolloids, flour, salts, malt flour, malt extract, gluten and starch.

## Patentansprüche

1. Eine Zusammensetzung umfassend Körnchen, geeignet zur Verwendung bei der Herstellung eines Teigs, wobei die Körnchen einen mittleren Durchmesser im Bereich von 30-500 µm aufweisen und umfassen:
a. einen hydrophilen Kern mit einem Durchmesser von mindestens 5 µm, wobei der Kern ein oder mehrere Enzyme enthält; und
b. eine lipophile, im Wesentlichen kontinuierliche Schicht, die den Kern einkapselt, wobei die Schicht mindestens 50 Gew.-% eines Triglycerid-Fetts mit einem Steigschmelzpunkt von mindestens 30 °C und mindestens 1 Gew.-% eines Trennmittels, ausgewählt aus der Gruppe aus Monoglyceriden, Diacetylweinsäureester von Mono- und/oder Diglyerid (Datem), Stearyl-Lactylaten und Kombinationen davon, umfasst;
und ferner umfassend Backzutaten in teilchenförmiger Form, wobei die Backzutaten ausgewählt sind aus der Gruppe bestehend aus Redoxmitteln, Emulgiermitteln, Hydrokolloiden, Mehl, Salzen, Malzmehl, Malzextrakt, Gluten und Stärke;
wobei die Zusammensetzung ein freifließendes Verhalten aufweist.

2. Die Zusammensetzung gemäß Anspruch 1, wobei der Kern ein Enzym enthält, ausgewählt aus der Gruppe bestehend aus α-Amylase, β-Amylase, Xylanase, Hemi-Cellulase, Cellulase, Lipase, Protease, Glukoseoxidase, Oxidoreduktase, Lipoxygenase, Peroxidase, Ferulasäure-Esterase, Pullulanase, Invertase, Mannanase, Galaktomannanase, Laktase und Kombinationen davon.

3. Die Zusammensetzung gemäß Anspruch 1, wobei das Trennmittel Monoglycerid ist.

4. Die Zusammensetzung gemäß Anspruch 1, wobei das Trennmittel Datem ist.

5. Die Zusammensetzung gemäß einem der vorherigen Ansprüche, wobei die lipophile Schicht zwischen 2 und 40 Gew.-% des Trennmittels enthält.

6. Die Zusammensetzung gemäß einem der vorherigen Ansprüche, wobei das Triglycerid-Fett einen Steigschmelzpunkt im Bereich von 30 - 40 °C aufweist.

7. Die Zusammensetzung gemäß einem der vorherigen Ansprüche, wobei das Triglycerid-Fett ein N-Profil von N₂₀ > 50; 10 ≤ N₃₀ ≤ 60; und N₄₀ < 5 aufweist.

8. Die Zusammensetzung gemäß einem der vorherigen Ansprüche, wobei der mittlere Durchmesser der Körnchen im Bereich von 60 - 400 µm liegt.

9. Verwendung der Zusammensetzung gemäß einem der vorherigen Ansprüche zur Herstellung eines Teigs, bevorzugt eines Brotteigs.

10. Ein Verfahren zur Herstellung einer Zusammensetzung gemäß einem der Ansprüche 1 bis 8, wobei das Verfahren die Schritte umfasst:
a. Herstellen einer Vielzahl von Teilchen mit einem Durchmesser von mindestens 5 µm, wobei die Teilchen ein Enzym enthalten;
b. Herstellen eines Gemischs, umfassend mindestens 50 Gew.-% eines Triglycerid-Fetts mit einem Steigschmelzpunkt von mindestens 30 °C und mindestens 1 Gew.-% eines Trennmittels, ausgewählt aus der Gruppe aus Monoglyceriden, Diacetylweinsäureester von Mono- und/oder Diglyerid (Datem), Stearyl-Lactylaten und Kombinationen davon;
c. Sprühen des Gemischs, das in Schritt b. erhalten wurde, in geschmolzener Form, auf die Vielzahl von Teilchen, die in Schritt a. erhalten wurden, um eine Einkapselung der Teilchen mit einer im Wesentlichen kontinuierlichen Schicht aus dem Gemisch zu erreichen; und
d. Abkühlen der resultierenden eingekapselten Teilchen, um eine Vielzahl von eingekapselten Teilchen, welche Rieselfähigkeit aufweisen, zu erhalten; und
e. Einarbeiten von ein oder mehr Backzutaten in teilchenförmiger Form, wobei die eine oder mehr Backzutaten ausgewählt sind aus der Gruppe bestehend aus Redoxmitteln, Emulgiermitteln, Hydrokolloiden, Mehl, Salzen, Malzmehl, Malzextrakt, Gluten und Stärke.

11. Ein Verfahren zur Herstellung einer Zusammensetzung gemäß einem der Ansprüche 1 bis 8, wobei das Verfahren die Schritte umfasst:
a. Herstellen einer Vielzahl von Teilchen mit einem Durchmesser von mindestens 5 µm, wobei die Teilchen ein Enzym enthalten;
b. Mischen der Vielzahl von Teilchen mit Triglycerid-Fett und einem Trennmittel, ausgewählt aus der Gruppe aus Monoglyceriden, Diacetylweinsäureester von Mono- und/oder Diglyerid (Datem), Stearyl-Lactylaten und Kombinationen davon, um ein Gemisch zu erhalten, wobei der lipophile Bestandteil mindestens 50 Gew.-% an Triglycerid-Fett mit einem Steigschmelzpunkt von mindestens 30 °C und mindestens 1 Gew.-% des Trennmittels enthält;
c. Herstellen einer homogenen Suspension aus dem Gemisch das in Schritt b. erhalten wird, wobei die kontinuierliche Phase der Suspension durch den geschmolzenen lipophilen Bestandteil gebildet wird;
d. Atomisieren der homogenen Suspension zu einem gasförmigen oder flüssigen Medium mit einer Temperatur unter dem Schmelzpunkt des lipophilen Bestandteils; und
e. Rückgewinnen der resultierenden Körnchen; und
f. Einarbeiten von einer oder mehr Backzutaten in teilchenförmiger Form, wobei die eine oder mehr Backzutaten ausgewählt sind aus der Gruppe bestehend aus Redoxmitteln, Emulgiermitteln, Hydrokolloiden, Mehl, Salzen, Malzmehl, Malzextrakt, Gluten und Stärke.

## Revendications

1. Composition comprenant des granulés aptes à être utilisés dans la préparation d'une pâte, lesdits granulés ayant un diamètre moyen dans la gamme de 30-500 µm et comprenant :
a. un noyau hydrophile présentant un diamètre d'au moins 5 µm, ledit noyau contenant une ou plusieurs enzymes; et
b. une couche lipophile sensiblement continue encapsulant le noyau, laquelle couche contient au moins 50 % en poids de triglycérides dont le point d'écoulement est d'au moins 30°C et au moins 1 % en poids d'un agent de libération sélectionné dans le groupe des monoglycérides, ester diacétyl-tartrique de mono et/ou de diglycéride (datem), stéaryl-lactylates et combinaisons de ceux-ci;
et comprenant en plus ingrédients de boulangerie sous forme particulaire, lesdits ingrédients de boulangerie étant choisis dans le groupe consistant en des agents redox, émulsifiants, hydrocolloïdes, farine, sels, farine de malt, extrait de malt, gluten et amidon;
ladite composition présentant un comportement d'écoulement.

2. Composition selon la revendication 1, dans laquelle le noyau contient une enzyme choisie dans le groupe consistant en l'α-amylase, β-amylase, xylanase, hémi-cellulase, cellulase, lipase, protéase, glucose oxydase, oxydoréductase, lipoxygénase, péroxidase, acide férulique estérase, pullulanase, invertase, mannanase, galactomannanase, lactase et combinaisons de ceux-ci.

3. Composition selon la revendication 1, dans laquelle l'agent de libération est le monoglycéride.

4. Composition selon la revendication 1, dans laquelle l'agent de libération est le datem.

5. Composition selon l'une quelconque des revendications précédentes, dans laquelle la couche lipophile contient entre 2 et 40 % en poids d'agent de libération.

6. Composition selon l'une quelconque des revendications précédentes, dans laquelle le triglycéride présente un point d'écoulement dans la gamme 30-40°C.

7. Composition selon l'une quelconque des revendications précédentes, dans laquelle le triglycéride présente un indice N de N₂₀ > 50 ; 10 ≤ N₃₀ ≤ 60 ; et N₄₀ < 5.

8. Composition selon l'une quelconque des revendications précédentes, dans laquelle le diamètre moyen des granulés est dans la gamme 60-400 µm.

9. Utilisation de la composition selon l'une quelconque des revendications précédentes dans la préparation d'une pâte, de préférence une pâte à pain.

10. Un procédé de fabrication d'une composition selon l'une quelconque des revendications 1 à 8, ledit procédé comprenant les étapes de :
a. préparation d'une pluralité de particules avec un diamètre moyen d'au moins 5 µm, lesdites particules contenant de l'enzyme ;
b. préparation d'un mélange contenant au moins 50 % en poids de triglycéride avec un point d'écoulement d'au moins 30°C et au moins 1% en poids d'un agent de libération choisi dans le groupe des monoglycérides, ester diacétyl-tartrique de mono et/ou de diglycéride (datem), stéaryl-lactylates et combinaisons de ceux-ci; et
c. pulvérisation du mélange obtenu à l'étape b. sous forme fondue sur la pluralité de particules obtenues à l'étape a. pour réaliser l'encapsulation des particules avec une couche sensiblement continue dudit mélange;
d. refroidissement des particules encapsulées résultantes pour obtenir une pluralité de particules encapsulées qui présentent un comportement d'écoulement; et
e. incorporation d'un ou plusieurs ingrédients de boulangerie sous forme particulaire, lesdits un ou plusieurs ingrédients de boulangerie étant choisis dans le groupe consistant en des agents redox, émulsifiants, hydrocolloïdes, farine, sels, farine de malt, extrait de malt, gluten et amidon.

11. Procédé de fabrication d'une composition selon l'une quelconque des revendications 1 à 8, ledit procédé comprenant les étapes de :
a. préparation d'une pluralité de particules avec un diamètre moyen d'au moins 5 µm, lesdites particules contenant de l'enzyme;
b. combinaison de la pluralité de particules avec des triglycérides et un agent de libération choisi dans le groupe des monoglycérides, ester diacétyl-tartrique de mono et/ou de diglycéride (datem), stéaryl-lactylates et les combinaisons de ceux-ci pour donner un mélange dans lequel le composant lipophile contient au moins 50 % en poids de triglycéride avec un point d'écoulement d'au moins 30°C et au moins 1% en poids d'agent de libération;
c. préparation d'une suspension homogène à partir du mélange obtenu à l'étape b., dans lequel la phase continue de la suspension est formée d'un composant lipophile fondu;
d. vaporisation de la suspension homogène dans un milieu gazeux ou liquide avec une température inférieure au point de fusion du composant lipophile; et
e. récupération des granulés résultants; et
f. incorporation d'un ou plusieurs ingrédients de boulangerie sous forme particulaire, lesdits un ou plusieurs ingrédients de boulangerie étant choisis dans le groupe consistant en des agents redox, émulsifiants, hydrocolloïdes, farine, sels, farine de malt, extrait de malt, gluten et amidon.
